# NEW EUROPEAN PATENT SPECIFICATION

(11) **EP 0 572 942 B2**
(45) Date of publication and mention of the opposition decision: **29.11.2000**
(45) Mention of the grant of the patent: 09.04.1997
(21) Application number: 93108658.1
(22) Date of filing: 28.05.1993
(51) Int. Cl.: A61K 9/28, A61K 9/20, A61K 9/54, A61K 9/50

(54) **Oral pharmaceutical compositions for specific colon delivery**
Orale Arzneimittel mit kolonspezifischer Freisetzung
Préparations pharmaceutiques orales à délivrance spécifique dans le colon

(30) Priority: 01.06.1992 IT MI921347
(43) Date of publication of application: 08.12.1993
(73) Proprietor: Monsanto Italiana S.p.A., 20068 Peschiera Borromeo (IT); POLICHEM S.A., Luxembourg (LU)
(72) Inventor: Poli, Stefano, I-20089 Quinto de' Stampi-Rozzano, (MI) (IT); Busetti, Cesare, I-20089 Quinto de' Stampi-Rozzano, (MI) (IT); Moro, Luigi, I-20089 Quinto de' Stampi-Rozzano, (MI) (IT)
(74) Representative: Grünecker, Kinkeldey, Stockmair & Schwanhäusser Anwaltssozietät

(56) References cited:
- EP-A- 0 305 918
- EP-A- 0 366 621
- EP-A- 0 384 514
- EP-A- 0 421 921
- EP-A- 0 453 001
- WO-A-91/04015
- WO-A-91/16042
- US-A- 5 096 717
- Davis, S.S. et al, Gut, 27, p. 886-892 (1986)
- Leopold C.S. and Eikeler D., Proc. 2nd World Meeting APGI/APV, Paris, 25/28 May 1998, p. 363-364
- Pschyrembel Klinisches Wörterbuch, 255. Auflage, p. 323

## Description

The present invention refers to delayed release oral pharmaceutical compositions for specific colon delivery.

The selective release of drugs in the colon has recently become more and more important in view of a number of therapeutic applications such as the oral administration of proteins and peptides, the treatment of bacterial infections of colon, the administration of anti-tumor, anti-inflammatory or antimycotic agents.

The systems up-to-now used may be generally classified as relying either on a chemical approach or on a technological approach, the former consisting in the use of pro-drugs and the latter in the use of biodegradable polymers or polymers with pH-dependent solubility.

The different approach on which the present invention is based consists in the possibility of exploiting the remarkable regularity of the transit time of the small intestine (3±1 hours according to Davis S.S. et al., Gut, 27 (1986) 886): the system proposed relies on the use of a control mechanism which can recognize the entry into the small intestine and of a polymer or polymers or copolymers mixture preventing the drug release for the time needed to transit through the small intestine segment.

EP-A-0 421 921 discloses pharmaceutical compositions consisting of a core containing disodium pamidronate, an hydrophilic elastic intermediate layer and an outer gastroresistant layer. The intermediate hydrophilic elastic layer is applied on the core with a maximum weight gain of 8% and has the purpose to avoid the contact of the gastroresistant layer with the active ingredient.

EP-A-0 453 001 discloses an extended release system, also capable of releasing an active ingredient in the colon tract, formed by a core containing the active ingredient, a membrane being soluble at pH equal to or greater than 5.5, a membrane being insoluble at said pH but permeable to intestinal fluids. The weight gain with respect to the core, although not explicitly stated, is of about 2%. The system disclosed in this document does not have a lag-time, during which no release of the active principle occurs, but, once entered in the intestinal tract, and once pH has risen over 5.5, the release occurs slowly and constantly throughout the intestine, with particular release in the colon.

EP-A-0 366 621 discloses a similar colon selective delivery system including a core of a therapeutically active substance coated by three different layers: an inner layer including an anionic polymer, an outer gastroresistant layer and an intermediate swellable layer constituted by high viscosity cellulose.

This intermediate layer is constituted by cellulose derivatives with high molecular weight.

The aqueous solutions of these substances present a high viscosity, which strongly requires to use an organic solvent (in which the cellulose derivative is practically insoluble) for the application of the intermediate polymeric layer, in order to maintain acceptable the range of process cost and time.

We have surprisingly noticed that it is anyhow possible to obtain a considerable release delay using a low viscosity cellulose derivative film.

These derivatives show some advantages in respect of the ones of the mentioned patent, among which the most important is that their water solutions, at the concentrations suitable for film coating process, have a remarkable lower viscosity and therefore the use of filmogenic aqueous solutions is allowable; as certainly known, this represent a considerable advantage from the ecological and environmental point of view in respect to the use of organic solvents.

Furthermore the use of low viscosity polymers, characterized by a quicker solubilization or erosion time, allows to maintain at thinner levels the gel layer generated upon hydration, this allows to obtain a higher speed of release (improved burst effect, like a spike), when the desired delay time is reached.

The present invention refers to a core surrounded by only two layers of polymers, whereas the patent EP-A-0-366 621 is referred to a three layers system and, consequently, it needs a higher number of manufacturing steps, with the obvious rebounds on the production costs.

Moreover the cited patent describes a pH-dependent system, because the drug release is ultimately limited (after the delay due to the intermediate layer) by the inner layer, which must be dissolved to allow the drug to be released: this inner layer is in any case constituted by a polymer soluble only at a pH value equal or more than 7.

The present invention allows the following advantages to be reached:
1) a more flowable process because one layering step has been removed;
2) the achievement of a potential more rapid release after the programmed lag-phase (more bursting release slope);
3) a completely pH-independent release pattern after the system activation.

Moreover, a further advantage of this application should be considered the suitability of low viscosity polymers; in fact this allows to design an aqueous coating procedure instead of an organic solvent, where environmental and antipollution policy have to be respected. If the costs of waste treatment should be considered, an effective saving is so obviously achievable.

Of course, where necessary, an organic polymer dispersion can be advantageously used.

The compositions of the present invention, that is easier to manufacture and cheaper than the prior art systems, includes:
a) a core containing the active principle or a mixture of active principles, optionally combined with excipients;
b) an intermediate coating layer of said core, including a hydrophilic swellable polymer or copolymer or mixture thereof, whose swelling/dissolution/erosion can delay the release of the active principle contained in the core for the programmed time, independently of the pH;
c) an outer layer whose dissolution activates the process of swelling/dissolution/erosion of the intermediate coating layer of point b),
characterized in that the intermediate coating layer is applied on the core until a weight gain, determined as solid substance, ranging between 10 and 300%, is reached, said compositions having a completely pH-independent release pattern after the system activation.

For instance the activation starts by "recognizing" the exit of the system from the stomach and the entry into the small intestine. The trigger point is represented by the change of pH after the pylorus.

The core containing the active principle may be differently formulated (tablets, capsules, soft capsules, mini-tablets, granules, pellets, spheronized crystals), so as to allow the prompt release of the active agent or a further controlled release, after the desired lag-phase. For this purpose, core excipient either conventional either able to form a matrix system may be used. Said core may also consist of the active principle alone, in crystalline or amorphous form.

In a typical embodiment, the intermediate coating layer includes a hydrophylic gelling polymer or copolymer (swelling polymer) or mixture thereof starting its swelling when the system is activated (triggered) by the dissolution of the outer gastroresistant layer. The swelled layer surrounding the core must have suitable characteristics to grant the complete protection of the substances contained in the core from biological fluids for the time needed to reach the release site.

The used polymers usually include methylcellulose, carboxymethylcellulose, hydroxypropylcellulose, hydroxypropylmethylcellulose, carbomers, polyvinyl alcohols, polyoxyethylene glycols, polyvinylpyrrolidones, poloxamers, natural or synthetic rubbers, polysaccharides or derivatives or mixtures thereof.

Besides the so called swellable polymers, other kinds of substances such as polysaccharides, polyaminoacids, polyalcohols, polyglycols or other ones having the capability of dissolving or becoming freely permeable with an exactly defined kinetic following hydration in aqueous fluids may be effectively used according to this invention. Examples of these substances are provided by gelatine, saccharose, sorbitol, mannanes, jaluronic acid or its synthetic derivatives or the like. The polymer may be applied on the core by different techniques, such as the spray coating or double tabletting (press-coating).

The hydrophilic intermediate polymeric layer is applied on the core until a weight gain, determined as solid substance, ranging between 10 and 300%, preferably between 50 and 150%, is reached.

In a preferred embodiment, the intermediate coating layer is composed, as unique or main component, by a cellulose derivative, preferably a hydroxypropylmethylcellulose giving a 2% aqueous solution with viscosity ranging from 5 to 100 mPa.s at room temperature.

The intermediate coating layer can further contain conventional auxiliary substances, such as hydrophobic materials like lubricants, flow promoting agents, plasticizers and antisticking agents. Examples of auxiliary substances are polyethylene glycol, polyvinylpyrrolidone, talc, magnesium stearate, glyceryl behenate, stearic acid, titanium dioxide.

The outer layer includes a material whose dissolution is pH-dependent.

The gastroresistant outer layer may be obtained by using the polymers usually available to this purpose such as cellulose acetophthalate, cellulose acetate terephtalate, cellulose acetate trimellitate, hydroxypropylmethylcellulose phthalate, polyvinyl alcohol phthalate, polyacrylate or polymethacrylates.

The outer layer may optionally includes hard or soft gelatine wall (capsules) coated by the above described gastroresistant polymers (i.e. the system consists of gastroresistant capsules filled with delayed release units).

Examples of active principles which may be used according to the invention comprise peptide or protein substances, ergot alkaloids or combinations thereof, hormone substances or their precursors, substances endowed with antimicrobial activity, like antiprotozoarian, antibacterial or antimycotic substances, immunomodulating, immunostimolant or immunosuppressive agents, vaccines, peptide hormone analogues or antagonists. Specific examples of these substances comprise posatirelin, protirelin, calcitonin, insulin, glutathione, hematopoietine, hirudin, cyclosporin, interferon, desmopressin, thymopentin, pidotimod.

It is comprised in the present invention a process for the preparation of the oral pharmaceutical compositions above described. Said process comprises the steps of
a) forming a core of active principle or of a mixture of active principles, optionally combined with excipients;
b) applying an intermediate coating layer on said core by means of a film coating or press-coating processes;
c) applying an outer layer on said intermediate coating layer.

In a preferred embodiment, the intermediate coating layer as to point b) is applied on said core until a weight gain, determined as solid substance, ranging from 10 to 300%, preferably from 50 to 150%, is reached.

The intermediate layer is applied on the core by means of a film coating process, either in a fluid bed apparatus or in a pan coat, or a atomization process, or a press coating process.

In a more preferred embodiment, the intermediate layer is applied on the core by means of a film coating process, by spraying an aqueous polymeric dispersion or an organic or hydro-organic solvent polymeric dispersion with a solid content ranging between 2 and 50% w/w, preferably ranging between 5 and 25 %.

The following non limitative examples further illustrate the invention.

In the examples, dissolution test was carried out according to the following standard method:
Acidic medium: 900 ml HCl 0.01 N
Neutral medium: 900 ml phosphate buffer pH 7.5
Temperature: 37°C
Apparatus: paddle (number 2 - according to USP XXII ed.) 50 RPM.

### EXAMPLE 1

Tablets containing 20 mg of posatirelin are formulated by using an excipient mixture consisting of lactose, microcrystalline cellulose, polyvinylpyrrolidone and magnesium stearate. The tablets, obtained by direct compression, are fluid-bed coated with a Methocel E50-LV^{(TM)} (showing a viscosity as high as 50 mPa.s at 2% aqueous solution) containing PEG as plasticizer. After intermediate layer deposition is completed, the tablets are made gastroresistant by applying a further coating layer of cellulose acetate phthalate containing diethylphthalate as plasticizer.

The dissolution test carried out on the finished systems showed the absence of release at pH lower than 5 for at least 2 hours. Upon increasing the pH of the dissolution medium up to 7.5 no drug release occours for the subsequent 4 hours; then 70% of the active agent is released within 45 minutes.

### EXAMPLE 2

Tablet cores containing 4 mg of pidotimod are formulated using an excipient mixture consisting of dibasic calcium phosphate, microcrystalline cellulose, glyceryl behenate and silicon dioxide.

The tablets are then charged into a tabletting apparatus having three feeding stations, respectively filled with a mixture of carboxyvinylpolymer, polyvinylpyrrolidone and polyethylenglycol the first, with said cores the second one and with the cited polymer mixture of the first station the third one.

The obtained small diameter coated tablets are distributed into gelatine capsules which in turn are covered with a methacrylic ester suspension.

The drug release pattern of these systems, determined as above specified, shows the absence of drug release for the first 3 hours subsequent the change of pH medium (trigger point).

### EXAMPLE 3

Tablet cores (180 mg weight, 6.7 mm diameter) containing 10 mg of posatireline are formulated using an excipient mixture consisting of dibasic calcium phosphate, microcrystalline cellulose, sodium starch glycolate and magnesium stearate. The tablets, obtained by direct compression, show a complete drug dissolution within 5 minutes.

The tablets are coated in an automatic pan with an aqueous solution of Methocel E15-LV^{(TM).} The solution contains PEG as plasticizer. Samples having different weight gains have been collected for examination at increasing times.

Subsequently the systems are coated again with an aqueous gastroresistant suspension of CAP, containing triacetine as plasticizer.

Dissolution tests performed on the collected samples have shown a good correlationship between weight gain relevant to intermediate coating layer and lag release time after activation:

| WEIGHT GAINS after first coating | LAG-PHASE (MINUTES) |
|---|---|
| 10% | 10 |
| 20% | 25 |
| 30% | 35 |
| 40% | 50 |
| 50% | 70 |
| 70% | 90 |
| 100% | 130 |
| 125% | 160 |
| 150% | 200 |

### EXAMPLE 4

Tablets of 180 mg weight containing 100 I.U. of salmon calcitonin, have been prepared; methylparaben has been used as release marker.

The tablets are press-coated in a tabletting unit with a low viscosity hydroxypropylmethylcellulose (Methocel E15-LV^{(T.M.)}; polyvinylpyrrolidone and magnesium stearate have been used as auxiliary substances. Subsequently the tablets are coated with an aqueous gastroresistant suspension of methacrylic ester suspension.

The release pattern has been demonstrated dependent on the HPMC type: in fact, taking constant the thickness of the coating all around (1.2 - 1.8 mm) a delay time of 100 minutes in achieving detectable levels of the marker has been obtained

### EXAMPLE 5

Operating as described in the example 4, has been changed the polymer type of the intermediate layer: by using Methocel E50-LV^{(T.M.)} a release delay of more than 160 minutes in the same conditions has been obtained.

### EXAMPLE 6

Operating as described in the example 3, tablet cores containing 3.000.000 I.U. of natural extractive alfa-interferon and 5 mg of methylparaben as marker has been coated by spraying a hydroalcoholic (water - ethyl alcohol) dispersion of Methocel^{(TM)} E50-LV at a 10% concentration containing also 5% of magnesium stearate, 2% of PVP and 1% of PEG 6000. A gastroresistant layer is then applied with the known method.

No drug release was observed during the first 2 hours in acidic medium and for at least 3 hours after the system activation.

## Claims

1. Oral pharmaceutical compositions including
a) a core containing the active principle or a mixture of active principles optionally combined with excipients;
b) an intermediate coating layer of said core, including a hydrophilic swellable polymer or copolymer or mixtures thereof, whose swelling/dissolution/erosion can delay the release of the active principle contained in the core for the programmed time, independently of the pH;
c) an outer layer whose dissolution activates the process of swelling/dissolution/erosion of the intermediate coating layer of point b);
characterized in that
the intermediate coating layer is applied on the core until a weight gain, determined as solid substance, ranging between 10 to 300%, is reached,
said compositions having a completely pH-independent release pattern after the system activation.

2. Compositions according to claim 1, wherein the outer layer described in point c) includes a material whose dissolution is promoted by the change of pH associated with the passage through the pylorous.

3. Compositions according to claim 2, wherein the hydrophilic polymer is selected from methylcellulose, carboxymethylcellulose, hydroxypropylcellulose, hydroxypropylmethylcellulose, carbomers, polyvinyl alcohols, polyoxyethylene glycols, polyvinylpyrrolidones, poloxamers, natural or synthetic rubbers, polysaccharides or derivatives or mixtures thereof.

4. Compositions according to claim 3, wherein the intermediate coating layer is applied on the core until a weight gain, determined as solid substance, ranging between 50 and 150%, is reached.

5. Compositions according to claim 4, wherein the intermediate coating layer is composed, as unique or main component, by a cellulose derivative, preferably a hydroxypropylmethylcellulose giving a 2% aqueous solution with a viscosity ranging from 5 to 100 mPa.s at room temperature.

6. Compositions according to claim 5, wherein the intermediate coating layer contains conventional auxiliary substances, such as hydrophobic materials like lubricants, flow promoting agents, plasticizers and antisticking agents.

7. Compositions according to claim 6, wherein the auxiliary substances for the intermediate coating layer are selected from the group including polyethylene glycol, polyvinylpyrrolidone, talc, magnesium stearate, glyceryl behenate, stearic acid, titanium dioxide, triacetine, silicon dioxide.

8. Compositions according to any one of the preceding claims, wherein the outer layer includes gastroresistant polymers or copolymers or mixtures thereof, preferably cellulose acetate phthalate or polymethacrylic derivatives.

9. Compositions according to any one the preceding claims, wherein the outer layer includes hard or soft gelatine wall (capsules) coated with gastroresistant polymers or copolymers.

10. Compositions according to any one of the preceding claims, wherein the active ingredient is posatirelin, protirelin, calcitonin, insulin, glutathione, hematopoietin, hirudin, cyclosporin, interferon, desmopressin, thymopentin, pidotimod.

11. A process for the preparation of oral pharmaceutical compositions of claims 1-10 comprising the steps of
a) forming a core of active principle or of a mixture of active principles, optionally combined with excipients;
b) applying an intermediate coating layer on said core by means of a film coating or press-coating processes;
c) applying an outer layer on said intermediate coating layer.

12. A process according to claim 11, wherein said intermediate coating layer as to point b) is applied on said core until a weight gain, determined as solid substance, ranging from 10 to 300%, preferably from 50 to 150%, is reached.

13. A process according to claims 11-12, wherein said intermediate layer is applied on the core by means of a film coating process, either in a fluid bed apparatus or in a pan coat, or a atomization process, or a press coating process.

14. A process according to claims 11-12, wherein the intermediate layer is applied on the core by means of a film coating process, by spraying an aqueous polymeric dispersion or an organic or hydro-organic solvent polymeric dispersion with a solid content ranging between 2 and 50% w/w, preferably ranging between 5 and 25%.

## Patentansprüche

1. Orale pharmazeutische Zusammensetzungen, umfassend:
a) einen Kern, der den Wirkstoff oder ein Wirkstoffgemisch, gegebenenfalls in Kombination mit Exzipientien, enthält,
b) eine Zwischenbeschichtungsschicht des Kerns, die ein hydrophiles, quellbares Polymer oder Copolymer oder Gemische davon umfasst, deren Quellung/Auflösung/Erosion die Freigabe des im Kern enthaltenen Wirkstoffs für die programmierte Zeit unabhängig von dem pH-Wert verzögern kann,
c) eine Außenschicht, deren Auflösung den Prozess der Quellung/Auflösung/Erosion der Zwischenbeschichtungsschicht von Punkt b) aktiviert,
**dadurch gekennzeichnet,** dass die Zwischenbeschichtungsschicht auf den Kern aufgebracht wird bis eine Gewichtszunahme, bestimmt als Festsubstanz, im Bereich zwischen 10 und 300 % erreicht ist, wobei die Zusammensetzungen ein völlig pH-unabhängiges Freigabemuster nach der Aktivierung des Systems aufweisen.

2. Zusammensetzungen nach Anspruch 1, worin die in Punkt c) beschriebene Außenschicht ein Material umfasst, dessen Auflösung durch die mit der Passage durch den Pylorus verbundene Veränderung des pH-Werts gefördert wird.

3. Zusammensetzungen nach Anspruch 2, worin das hydrophile Polymer aus Methylcellulose, Carboxymethylcellulose, Hydroxypropylcellulose, Hydroxypropylmethylcellulose, Carbomeren, Polyvinylalkoholen, Polyoxyethylenglykolen, Polyvinylpyrrolidonen, Poloxameren, natürlichen oder synthetischen Kautschuken, Polysacchariden oder Derivaten oder Gemischen davon ausgewählt ist.

4. Zusammensetzungen nach Anspruch 3, worin die Zwischenbeschichtungsschicht auf den Kern aufgebracht wird bis eine Gewichtszunahme, bestimmt als Festsubstanz, im Bereich zwischen 50 und 150 % erreicht ist.

5. Zusammensetzungen nach Anspruch 4, worin die Zwischenbeschichtungsschicht aus einem Cellulosederivat, bevorzugt einer Hydroxypropylmethylcellulose, die eine 2 %ige wässrige Lösung mit einer Viskosität im Bereich von 5 bis 100 mPa · s bei Raumtemperatur ergibt, als Einzel- oder Hauptkomponente zusammengesetzt ist.

6. Zusammensetzungen nach Anspruch 5, worin die Zwischenbeschichtungsschicht herkömmliche Hilfssubstanzen wie hydrophobe Materialien, wie Gleitmittel, Fließverbesserer, Weichmacher und Antiklebemittel enthält.

7. Zusammensetzungen nach Anspruch 6, worin die Hilfssubstanzen für die Zwischenbeschichtungsschicht aus der Gruppe umfassend Polyethylenglykol, Polyvinylpyrrolidon, Talk, Magnesiumstearat, Glycerylbehenat, Stearinsäure, Titandioxid, Triacetin, Siliciumdioxid, ausgewählt sind.

8. Zusammensetzungen nach einem der vorstehenden Ansprüche, worin die Außenschicht gastroresistente Polymere oder Copolymere oder Gemische davon, bevorzugt Celluloseacetatphthalat oder Polymethacrylsäurederivate, umfasst.

9. Zusammensetzungen nach einem der vorstehenden Ansprüche, worin die Außenschicht eine Hart- oder Weichgelatinewand (Kapseln), beschichtet mit gastroresistenten Polymeren oder Copolymeren, umfasst.

10. Zusammensetzungen nach einem der vorstehenden Ansprüche, worin der Wirkstoff Posatirelin, Protirelin, Calcitonin, Insulin, Glutathion, Hämatopoietin, Hirudin, Cyclosporin, Interferon, Desmopressin, Thymopentin, Pidotimod ist.

11. Verfahren zur Herstellung von oralen pharmazeutischen Zusammensetzungen nach den Ansprüchen 1 bis 10, umfassend die Stufen
a) Bilden eines Kerns aus dem Wirkstoff oder einem Wirkstoffgemisch, gegebenenfalls in Kombination mit Exzipientien,
b) Aufbringen einer Zwischenbeschichtungsschicht auf den Kern mittels eines Filmbeschichtungs- oder Druckbeschichtungsverfahrens,
c) Aufbringen einer Außenschicht auf die Zwischenbeschichtungsschicht.

12. Verfahren nach Anspruch 11, wobei die Zwischenbeschichtungsschicht gemäß Punkt b) auf den Kern aufgebracht wird bis eine Gewichtszunahme, bestimmt als Festsubstanz, im Bereich von 10 bis 300 %, bevorzugt von 50 bis 150 %, erreicht ist.

13. Verfahren nach den Ansprüchen 11 bis 12, wobei die Zwischenschicht auf den Kern mittels eines Filmbeschichtungsverfahrens entweder in einer Wirbelbettvorrichtung oder in einer Beschichtungspfanne oder mittels eines Atomisierungsverfahrens oder eines Druckbeschichtungsverfahrens aufgebracht wird.

14. Verfahren nach den Ansprüchen 11 bis 12, wobei die Zwischenschicht auf den Kern mittels eines Filmbeschichtungsverfahrens, durch Sprühen einer wässrigen Polymerdispersion oder einer Polymerdispersion mit einem organischen oder wässrig-organischen Lösungsmittel mit einem Feststoffgehalt im Bereich zwischen 2 und 50 % Gew./Gew., bevorzugt im Bereich zwischen 5 und 25 %, aufgebracht wird.

## Revendications

1. Compositions pharmaceutiques orales comprenant:
a) un coeur contenant le principe actif ou un mélange de principes actifs éventuellement combinés avec des excipients;
b) une couche de revêtement intermédiaire sur ledit coeur, comprenant un polymère ou un copolymère gonflable hydrophile ou un mélange de ceux-ci, dont les gonflement/dissolution/érosion peuvent retarder la libération du principe actif contenu dans le coeur pour la durée programmée, indépendamment du pH ;
c) une couche externe dont la dissolution active le processus de gonflement/dissolution/érosion de la couche de revêtement intermédiaire du point b); caractérisées en ce que la couche de revêtement intermédiaire est appliquée sur le coeur j'usqu'à ce qu'on obtienne un gain pondéral, déterminé par rapport aux substances solides, compris entre 10 et 300 %, lesdites compositions ayant un profil de libération complètement indépendant du pH après l'activation du système.

2. Compositions conformes à la revendication 1, dans lesquelles la couche externe décrite dans le point c) comprend un matériau dont la dissolution est activée par la modification de pH associée au passage par le pylore.

3. Compositions conformes à la revendication 2, dans lesquelles le polymère hydrophile est choisi parmi la méthylcellulose, la carboxyméthylcellulose, l'hydroxypropylcellulose, l'hydroxypropylméthylcellulose, des carbomères, des alcools polyvinylique, des polyoxyéthylèneglycols, des polyvinylpyrrolidones, des poloxamères, des caoutchoucs naturels ou synthétiques, des polysaccharides ou des dérivés ou des mélanges de ceux-ci.

4. Compositions conformes à la revendication 3, dans lesquelles la couche de revêtement intermédiaire est appliquée sur le coeur jusqu'à ce qu'on obtienne un gain pondéral, déterminé par rapport aux substances solides, compris entre 50 et 150 %.

5. Compositions conformes à la revendication 4, dans lesquelles la couche de revêtement intermédiaire est composée, comme composant unique ou principal, d'un dérivé de cellulose, de préférence une hydroxypropylméthylcellulose, donnant une solution aqueuse à 2 % ayant une viscosité comprise entre 5 et 100 mPa.s à température ambiante.

6. Compositions conformes à la revendication 5, dans lesquelles la couche de revêtement intermédiaire contient des substances auxiliaires classiques, telles que des matériaux hydrophobes tels que des lubrifiants, des agents favorisant l'écoulement, des plastifiants et des agents anti-adhérents.

7. Compositions conformes à la revendication 6, dans lesquelles les substances auxiliaires pour la couche de revêtement intermédiaire sont choisies dans l'ensemble comprenant le polyéthylèneglycol, la polyvinylpyrrolidone, le talc, le stéarate de magnésium, le béhénate de glycéryle, l'acide stéarique, le dioxyde de titane, la triacétine, le dioxyde de silicium.

8. Compositions conforme à l'une quelconque des revendications précédentes, dans lesquelles la couche externe comprend des polymères ou des copolymères gastro-résistants ou des mélanges de ceux-ci, de préférence de l'acétyl-phtalate de cellulose ou des dérivés polyméthacryliques.

9. Compositions conformes à l'une quelconque des revendications précédentes, dans lesquelles la couche externe comprend des parois (capsules) de gélatine dure ou molle revêtues de polymères ou de copolymères gastro-résistants.

10. Compositions conformes à l'une quelconque des revendications précédentes, dans lesquelles l'ingrédient actif est la posatiréline, la protiréline, la calcitonine, l'insuline, le glutathion, l'hématopoïétine, l'hirudine, la cyclosporine, l'interféron, la desmopressine, la thymopentine, le pidotimode.

11. Procédé de préparation de compositions pharmaceutiques orales des revendications 1-10 comprenant les étapes consistant à :
a) former un coeur de principe actif ou d'un mélange de principes actifs, éventuellement combinés à des excipients;
b) appliquer une couche de revêtement intermédiaire sur ledit coeur à l'aide de procédés d'enrobage ou de revêtement par compression ;
c) appliquer une couche externe sur ladite couche de revêtement intermédiaire.

12. Procédé conforme à la revendication 11, dans lequel ladite couche de revêtement intermédiaire du point b) est appliquée sur ledit coeur jusqu'à ce qu'on obtienne un gain pondéral, déterminé par rapport aux substances solides, compris entre 10 et 300 %, de préférence entre 50 et 150 %.

13. Procédé conforme aux revendications 11 et 12, dans lequel ladite couche intermédiaire est appliquée sur le coeur à l'aide d'un procédé d'enrobage, dans un appareil à lit fluidisé, ou par revêtement en bac, ou par un procédé d'atomisation, ou un procédé de revêtement par compression.

14. Procédé conforme aux revendications 11 et 12, dans lequel la couche intermédiaire est appliquée sur le coeur par un procédé d'enrobage, en pulvérisant une dispersion polymère aqueuse ou une dispersion polymère dans un solvant organique ou hydro-organique ayant une teneur en matières solides comprise entre 2 et 50 % p/p, de préférence entre 5 et 25 %.
